Europäisches Patentamt

European Patent Office

Office européen des brevets

(19)

(11)  EP 1 547 559 A1

(12)  **EUROPEAN PATENT APPLICATION**
published in accordance with Art. 158(3) EPC

(43) Date of publication:
29.06.2005  Bulletin 2005/26

(51) Int Cl.7: **A61F 13/511**, A61F 13/472,
A61F 5/455

(21) Application number: 03799095.9

(22) Date of filing: **29.08.2003**

(86) International application number:
**PCT/JP2003/011064**

(87) International publication number:
**WO 2004/030594 (15.04.2004 Gazette 2004/16)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority:  **03.10.2002  JP 2002291556**

(71) Applicant: **UNI-CHARM CORPORATION
Kawanoe-shi Ehime 799-0111 (JP)**

(72) Inventors:
• **MIZUTANI, Satoshi, Uni-Charm Corporation
Mitoyo-gun, Kagawa 769-1602 (JP)**
• **NODA, Yuki, Uni-Charm Corporation
Mitoyo-gun, Kagawa 769-1602 (JP)**
• **SAKAI, Akane, Uni-Charm Corporation
Mitoyo-gun, Kagawa 769-1602 (JP)**
• **TOMIDA, Katsushi, Uni-Charm Corporation
Mitoyo-gun, Kagawa 769-1602 (JP)**

(74) Representative: **Main, Malcolm Charles et al
Murgitroyd & Company
Scotland House
165-169 Scotland Street
Glasgow G5 8PL (GB)**

(54)  **FRONT FACE SHEET AND INTRALABIAL PAD**

(57)     The present invention relates to an interlabial pad, which is fitted between labia and absorbs menstrual blood and other body fluids, and a surface side sheet used in this interlabial pad. The surface side sheet is used in the interlabial pad that is fitted between labia and has a fiber aggregate having a wet shear strength with respect to labial inner walls of no less than 15cN/25mm and no more than 60cN/25mm when containing an artificial menstrual blood at a content of 800%, the artificial menstrual blood being a fluid composition comprising water, glycerin, sodium carboxymethylcellulose, sodium chloride, sodium bicarbonate, and a food dye preparation. According to the present invention, even when the shape of the labia changes due to movement of a wearer in a highly wet state after absorption of menstrual blood, since the shear force that arises between the labial inner walls and the interlabial pad can be resisted, positional deviation and falling off of the interlabial pad can be prevented. The interlabial pad can thus be fitted securely between the labia even without using an adhesive agent.

## Fig. 1 A

EP 1 547 559 A1

**Description**

**FIELD OF THE INVENTION**

The present invention relates to an interlabial pad, which is fitted between labia and absorbs menstrual blood and other body fluids, and a surface side sheet used in this interlabial pad.

**RELATED ART**

Conventionally, menstrual napkins and tampons have been known as menstrual products. Among these, with menstrual napkins, there was the possibility of leakage of menstrual blood from gaps resulting from the lowering of adhesion to the vicinity of the ostium vaginae. Meanwhile tampons tended to give rise to foreign body sensations and discomfort during use.

Interlabial pads have come to be noted recently as menstrual products that resolve the above issues by incorporating the advantages of menstrual napkins and tampons.

That is, an interlabial pad is held by being sandwiched by the labia, is high in adhesion to the body, and is thus less likely to give rise to leakage of menstrual blood in comparison to menstrual napkins and also low in terms of foreign body sensations and discomfort in comparison to tampons, which are inserted inside the vagina.

However, since an interlabial pad is held by being sandwiched by the labia, it had the possibility of falling off from between the labia when put in a highly wet state by the menstrual blood of a wearer. This is because menstrual blood becomes retained excessively at the interface of the labial inner walls and the surface of the interlabial pad opposing the labial inner walls and when this menstrual blood flows, the shear strength of the interlabial pad with respect to the labial inner walls is lowered.

The following two methods have been proposed to resolve this issue.

With a first method, an interlabial pad comprises a main absorbent body and a pair of absorbent flexible elongate parts that are provided at upper parts of the main absorbent body and maintain the state of contact with the labial inner walls (see for example, United States Patent No. 6033391 Specification). Here, a biocompatible adhesive agent is coated on the labial inner wall sides of the flexible elongate parts.

With this method, the flexible elongate parts are adhered to the labial inner walls by the biocompatible adhesive agent, so that falling off of the interlabial pad due to movement of the wearer can be prevented. Furthermore, even when the interlabial pad becomes highly wetted due to absorption of menstrual blood and other body fluids, the airtightness between the labial inner walls and the surface of the interlabial pad can be maintained by the adhesive strength of the biocompatible adhesive agent and leakage of menstrual blood can thus be prevented.

With a second method, a surface side sheet of an interlabial pad is formed by blending a biocompatible adhesive agent (for example, wax) with fibers. This adhesive agent softens at a lowly wet state of a water content of 400% or less.

With this method, when the surface side sheet is made to contact the inner surfaces of the labia, the biocompatible adhesive agent in the surface side sheet is wetted and softened by the body fluids of the wearer and adheres to the labial inner walls.

However, with the above-described first method, components of the biocompatible adhesive agent may elute out during wear and, depending on the body constitution of the wearer, may cause a rash or itching of genital parts. Also, depending on the amount of biocompatible adhesive agent coated, the permeability of the flexible elongate parts may become lowered and the menstrual blood may leak to the exterior without becoming absorbed by the main absorbent body.

Also with the above-described second method, when the interlabial pad is put in a highly wet state of a body fluid content of 600% or more, the biocompatible adhesive agent softens excessively and since an adequate adhesive strength cannot be provided, the interlabial pad may fall off. Furthermore, when the adhesive agent softens excessively, the fibers that make up the surface side sheet may be retained on the labial inner walls when the interlabial pad is removed.

**SUMMARY OF THE INVENTION**

The present invention has been made in view of issues such as the above and an object thereof is to provide a surface side sheet, which can be fitted securely between the labia even if an adhesive agent is not used, and an interlabial pad, which uses this surface side sheet.

In order to resolve issues such as the above, the present inventors noted that for prevention of fall-off of an interlabial pad from between the labia, adhesion of the labial inner walls with the interlabial pad's surface side sheet, which opposes the labial inner walls, that is, the strength in the direction of the shear between the labial inner wall

EP 1 547 559 A1

surfaces and the surface side sheet is important, and have come to complete the present invention.

More specifically, the present invention provides the following.

(1) A surface side sheet for being used in an interlabial pad fitted between labia, comprising: a fiber aggregate, wherein a wet shear strength of said fiber aggregate with respect to labial inner walls is no less than 15cN/25mm and no more than 60cN/25mm where containing an artificial menstrual blood at a content of 800%, the artificial menstrual blood is a fluid composition comprising water, glycerin, sodium carboxymethylcellulose, sodium chloride, sodium bicarbonate, and a food dye preparation.

Here, the shear strength is used to evaluate the adhesion of the interlabial pad with the labial inner walls when a wearer puts on the interlabial pad.

The surface side sheet is manufactured by a method such as spun bonding, point bonding, through air method, needle punching, wet paper method, wet forming spun lacing, etc.

The artificial menstrual blood is, for example, prepared by the following method using the following reagents and equipment.

<Reagents>

(A) Sodium carboxymethylcellulose (NaCMC): Wako Pure Chemical Industries Ltd., Chemical Grade 039-01335.
(B) Glycerin: Wako Pure Chemical Industries Ltd., Wako Primary Grade 072-00621
(C) Sodium chloride (NaCl): Wako Pure Chemical Industries Ltd., Special Reagent Grade 191-01665
(D) Sodium bicarbonate (NaHCO$_3$): Wako Pure Chemical Industries Ltd., Wako Primary Grade 198-01315
(E) Red food dye preparation: Koyo Products Co., Ltd., No. 102, Red No. 102
(F) Red food dye preparation: Koyo Products Co., Ltd., No. 2, Red No. 2
(G) Yellow food dye preparation: Koyo Products Co., Ltd., No. 5, Yellow No. 5

<Equipment>

(A) Pure water producing device: Yamato Scientific Co., Ltd., Pure Water Producing Device, Model WG220
(B) Stirrer: Hsiang Tai Machinery Industry Co., Ltd., Model DC-2E
(C) Viscometer: Shibaura Systems Co., Ltd., Vismetron, Model VGA-4

<Preparation Method>

320±2g of glycerin are placed in a plastic container 1, 32.0±0.3g of sodium carboxymethylcellulose (NaCMC) are added, and stirring at a rotation speed of approximately 600rpm is performed for 10 minutes with a stirrer to prepare a solution 1. Then while stirring 3 liters of ion-exchanged water placed in another plastic container 2 at a rotation speed of approximately 1100rpm using the abovementioned stirrer, the priorly prepared solution 1 is added by a small amount at a time. 1 liter of ion-exchanged water is added further while using it to rinse plastic container 1. 40g of sodium chloride (NaCl) and 16g of sodium bicarbonate (NaHCO$_3$) are then added by small amounts at a time while stirring to solution 2 obtained in the above manner, and after completion of adding, stirring is performed for approximately 3 hours. 32g, 8g, and 8g of the food dye preparations, Red No. 102, Red No. 2, and Yellow No. 5 (manufactured by Koyo Products Co., Ltd.), respectively, are then added while stirring to solution 3 prepared as described above and stirring is performed for approximately 1 hour thereafter to adjust the viscosity as measured by a viscometer to 22 to 26mPa•s.

The above-described artificial menstrual blood is made to be contained in the surface side sheet by the following procedures.

First, the artificial menstrual blood is sprayed in a mist-like form onto the surface side sheet until a predetermined content is reached and the entire surface side sheet is then covered with a liquid-impermeable material and left in this state for approximately 1 minute under an environment of 20°C and 60% humidity to make the artificial menstrual blood spread throughout the entire sheet.

Here, the content refers to the proportion of artificial menstrual blood absorbed by the surface side sheet and is defined by the following equation:

Content = Weight (g) of artificial menstrual blood sprayed onto the

3

surface side sheet / Weight (g) of the surface side sheet $\times$ 100

Since the environment of the labial inner walls varies greatly with the body constitution and health conditions of the wearer and the shapes of the labia also vary greatly in accompaniment with the movement of the wearer, the menstrual blood content in the surface side sheet varies greatly in a state in which the interlabial pad is fitted between the labia.

That is, a state in which the degree of wetting of the labial inner walls is low is a state prior to the discharge of menstrual blood and is a state of wetting by the mucus of the labial inner walls of the wearer, and the body fluid content in this state is approximately 300%.

On the other hand, a state in which the degree of wetting of the labial inner walls is high is a state in which the amount of menstrual blood discharged is high and the interlabial pad has been fitted for a long period of time, and in this state, the body fluid content is approximately 800%. In this case, even if a considerably high amount of menstrual blood is discharged, since the menstrual blood permeates into parts besides the surface side sheet or permeates into another menstrual product worn along with the interlabial pad, the body fluid content of the surface side sheet will not exceed 800%.

Since the body fluid content of the surface side sheet, which is a part of the interlabial pad, will thus be in a range of 300 to 800%, the adhesion of the labial inner walls with the interlabial pad will be the lowest in the state of the highest body fluid content of 800%.

According to the present invention, even when the shapes of the labia change due to movement of a wearer in a highly wet state after absorption of menstrual blood, since the shear force that arises between the labial inner walls and the interlabial pad can be resisted, positional deviation and falling off of the interlabial pad can be prevented. The interlabial pad can thus be fitted securely between the labia even without using an adhesive agent.

If the wet shear strength of the surface side sheet with respect to the labial inner walls is less than 15cN/25mm in a state where the artificial menstrual blood content is 800%, the interlabial pad may fall off due to the shear force that arises between the labial inner walls and the interlabial pad when the shape of the labia changes due to movement of the wearer. On the other hand, if the wet shear strength of the surface side sheet with respect to the labial inner walls is greater than 60cN/25mm in a state where the content of artificial menstrual blood is 800%, the labial inner walls may become damaged by the surface of the interlabial pad in the process of removing the interlabial pad.

The surface side sheet is preferably arranged from a fiber aggregate of hydrophilic fibers to which a hydrophilic surface active agent is not attached. With this arrangement, affinity with (wetting by) menstrual blood will be good, and the retention of menstrual blood at the interface of the labial inner walls and the surface side sheet and the lowering of the wet shear strength of the surface side sheet with respect to the labial inner walls due to the flowing of this menstrual blood will thus be prevented.

Also, the fiber aggregate that makes up the surface side sheet is preferably made loose in the binding force among fibers. By doing so, even when an impact is applied to the surface side sheet due to movement of the wearer, the impact can be cushioned by voids among the fibers due to the fibers being high in the degree of freedom, and the wet shear strength of the surface side sheet as a whole can thus be maintained.

The following methods may be considered for making the fiber aggregate, which makes up the surface side sheet, loose in the binding force among fibers. For example, there is a method wherein after forming the surface side sheet the sheet is subject to a tendering process or a crimp embossing process to distort the formed surface side sheet. There is also a method of arranging the surface side sheet itself from a fiber aggregate with which the binding force among fibers is loose.

Among such methods, a method of arranging the surface side sheet itself from a fiber aggregate with which the binding force among fibers is loose is preferable in terms of simplifying and stabilizing the manufacturing process. As the fiber aggregate, a spun-laced nonwoven fabric, obtained by using any one of various types of fibers solitarily or blending various types of fibers and then entangling the fibers by hydro-entanglement, is preferable. This is because though a surface active agent is attached to fibers in a fiber opening process in order to prevent non-uniformity of specific weight due to static electricity, this surface active agent can be removed substantially by water flow and chemical irritation due to the surface active agent can thus be reduced.

Here, hydro-entanglement refers to subjecting a web of deposited fibers to a high-pressure water jet to entangle the fibers.

By weakening the water pressure for entangling the fibers by water flow in the hydro-entanglement process, the binding force among fibers is loosened. For example, the water pressure is set to 50kg/cm$^2$ (converted value: 4.90MN/m$^2$) or less. Furthermore, if synthetic fibers are blended, the drying temperature in a drying process performed after hydro-entanglement is set to a temperature that is lower than the softening point of the synthetic fiber. Specifically, the surface side sheet is adjusted to a density in a range of 0.040 to 0.400g/cm$^3$ (converted value: 40 to 400kg/m$^3$)

and preferably in a range of 0.060 to 0.200g/cm$^3$ (converted value: 60 to 200kg/m$^3$).

The surface side sheet is preferably formed by layering fiber aggregates in which fibers with the same wetting property are blended.

For example, if the surface side sheet is formed with the proportion of hydrophilic fibers, such as pulp, natural cotton, rayon, acetate rayon, etc., being 70 to 95% and the proportion of synthetic fibers, such as polyethylene, polypropylene, polyethylene terephthalate being 30 to 5%, the absorptivity of absorption of menstrual blood will differ according to regions, and thus regions of high body fluid content and regions of low body fluid content may occur in the surface side sheet. Since the wet shear strength will thus differ according to regions within the surface side sheet, separation from the labial inner walls will occur at regions of low wet shear strength and, as a result, separation will occur at regions of high wet shear strength as well. The entire surface side sheet may thus separate and the interlabial pad may fall off.

(2) The above-described surface side sheet, wherein a bulk of said fiber aggregate in a wet state is no less than 80% of a bulk in a dry state.

Here, the dry state refers to a state wherein the surface side sheet is pressurized at 0.5g/cm$^2$ (converted value: 49N/m$^2$) after leaving for 2 hours at a temperature of 25 degrees and a humidity of 60%.

The wet state refers to a state wherein the surface side sheet is pressurized at 0.5g/cm$^2$ (converted value: 49N/m$^2$) after being wetted by making a predetermined amount of the above-described fluid composition (artificial menstrual blood) be contained.

According to the present invention, even if the content of menstrual blood in the surface side sheet becomes high, the surface in contact with the labial inner walls will not be immersed in menstrual blood. Since the fluidity of the menstrual blood can thus be restrained and the surface of contact with the labial inner walls can be maintained, the wet shear strength of the labial inner walls and the surface side sheet will not be lowered. Also, since the density of the surface side sheet will practically rise and the permeability of menstrual blood will weaken as the bulk decreases, leakage of menstrual blood will not occur.

(3) The above-described surface side sheet, wherein said fiber aggregate comprises: a surface layer formed of a hydrophilic fiber and a back layer formed of a hydrophilic fiber and a synthetic resin fiber.

Since synthetic resin fibers do not have a wetting property, the bulk can be kept nearly the same as that in the dry state even when wetted. Thus according to the present invention, by blending synthetic resin fibers with hydrophilic fibers, the bulk can be maintained even in a wet state. Thus even if the bulk of the surface layer cannot be kept at 80% or more in the wet state, the bulk of the surface side sheet as a whole can be kept at 80% or more of that of the dry state. Since retention of menstrual blood at the interface with the labial inner walls can thus be prevented, the wet shear strength of the surface side sheet with respect to the labial inner walls will not be lowered.

Here, the blended amount of the synthetic resin fibers is preferably no less than 6% and no more than 30%, and more preferably 10 to 20%. If the blended amount is less than 6%, the fiber layer of the back layer will not be able to maintain the bulk in the wet state and the bulk of the surface side sheet as a whole will be lowered. Also, when the blended amount is greater than 30%, the wetting property of the fiber layer of back layer will be poor and menstrual blood will not move readily to an absorbent body.

The hydrophilic fibers here are not restricted in particular, and pulp, natural cotton, rayon, and acetate rayon may be cited as examples. Meanwhile, as synthetic fibers, in addition to polyethylene (PE), polypropylene (PP), polyethylene terephthalate (PET), etc., fibers of crimped structure formed of heat-shrinkable composite fibers of PE/PP, PE/PET, PP/PP, etc. that have a core component of high melting point and a sheath component of low melting point, fibers, having a bulky structure due to having a hollow structure, etc., may be cited as examples.

(4) A surface side sheet for being used in an interlabial pad fitted between labia, comprising: a fiber aggregate having a tensile strength of no more than 600cN/25mm in a state where the surface side sheet contains an artificial menstrual blood at a content of 300% and is elongated by 5% in a longitudinal direction and having a tensile strength of no less than 100cN/25mm in a state where the surface side sheet contains said artificial menstrual blood at a content of 800% and is elongated by 5% in a longitudinal direction, the artificial menstrual blood is a fluid composition comprising water, glycerin, sodium carboxymethylcellulose, sodium chloride, sodium bicarbonate, and a food dye preparation.

Here, the tensile strength is that of the state of elongation by 5% instead of the state of breakage because whereas the tensile strength at breakage is due to the strength of the fibers that make up the surface side sheet, the tensile strength at 5% elongation is mainly due to the binding force among fibers.

According to the present invention, even when the surface side sheet absorbs menstrual blood and is put in a highly wet state, the binding force among the fibers that make up the surface side sheet will be loose and the degree of freedom among the respective fibers will be high. Thus even when an impact is applied to the surface side sheet due to movement of a wearer while the interlabial pad is fitted, this impact can be cushioned. Since the applied impact will thus hardly act as a shear force on the interface of the surface side sheet and the labial inner walls, positional deviation and falling off of the interlabial pad can be prevented. The interlabial pad can thus be fitted securely between the labia even without the use of an adhesive agent.

If the tensile strength is greater than 600cN/25mm in a state where the surface side sheet contains the artificial menstrual blood at a content of 300% and is elongated by 5% in the longitudinal direction, the binding force among the fibers will be too strong and the impact applied to the surface side sheet will reach the interface of the labial inner walls and the surface side sheet without being cushioned and cause the interlabial pad to fall off.

On the other hand, if the tensile strength is less than 100cN/25mm in a state where the surface side sheet contains the artificial menstrual blood at a content of 800% and is elongated by 5% in the longitudinal direction, the surface side sheet may break upon application of impact due to movement of the wearer and the interlabial pad may thus fall off. Furthermore, fibers that make up the surface side sheet may remain on the labial inner walls when the interlabial pad is removed.

The tensile strength in a state where the surface side sheet contains the artificial menstrual blood at a content of 800% and is elongated by 5% in the longitudinal direction is more preferably no more than 300cN/25mm and even more preferably no more than 200cN/25mm. When this tensile strength is greater than 300cN/25mm, the rigidity of the surface side sheet will be too high and the property of following the labial inner walls will be weak, and thus the wet tensile strength of the surface side sheet with respect to the labial inner walls in the wet state may decrease.

With regard to the rigidity of the surface side sheet, the B value (flexural rigidity per 1m width of cloth), measured at a curvature K of 0 to 2.0cm$^{-1}$, using the "KES Flexural Rigidity Tester," manufactured by Kato-Tech Co., Ltd., is preferably no more than $0.065 \times 10^{-4}$N·m$^2$/m and more preferably no more than $0.05 \times 10^{-4}$N·m$^2$/m, and the G value (shear rigidity per 1m width of cloth), measured at a shear angle of 0 to 5.0°, using the "KES Shear Tester" manufactured by the same maker, is preferably no more than 2.50N/m·degree and more preferably no more than 2.00N/m·degree.

The property of following the labial inner walls can thus be improved and the lowering of the wet shear strength of the labial inner walls and the surface side sheet in a highly wet state can be prevented. Also, even if impact due to movement of the wearer is applied, the surface side sheet will no be deformed by this impact, and even when a part of the surface side sheet becomes separated from the labial inner walls, falling off of the interlabial pad can be prevented.

(5) The above-described surface side sheet, wherein a Klemm absorption of said artificial menstrual blood for 10 minutes is no less than 10mm.

According to the present invention, the wetting property of the surface side sheet is set so that the Klemm absorption after 10 minutes, as measured in compliance with the "Water absorption testing method for paper and board by the Klemm method" of JIS P8141, is no less than 10mm. Menstrual blood is thus absorbed readily and, for example, the menstrual blood can be made to move to an absorbent body provided at the back side of the surface side sheet.

This Klemm absorption is preferably no less than 10mm and no more than 50mm. If the absorption is less than 10mm, the absorption of even a small amount of menstrual blood will be difficult. On the other hand, if the absorption is greater than 50mm, the surface side sheet itself will hold the menstrual blood and the menstrual blood will be retained at the interface of the surface side sheet and the labial inner walls, causing the wet shear strength of the surface side sheet with respect to the labial inner walls to decrease and the interlabial pad to fall off readily.

(6) A surface side sheet for being used in an interlabial pad fitted between labia, comprising: a fiber aggregate having a dense fiber distribution so as not to contact with labial inner walls non-uniformly.

If the fiber density in a surface side sheet is non-uniform, in a wet state in which menstrual blood is absorbed, the body fluids absorbed in regions of low density will move to regions of high density due to the density gradient and regions that differ in the content of menstrual blood will thus exist in the surface side sheet. The wet shear strength of the surface side sheet with respect to the labial inner walls will thus also be non-uniform and separation will occur readily at regions of low wet shear strength. As a result, separation will occur at regions of high wet shear strength as well, causing the entire surface side sheet to separate and the interlabial pad to fall off.

However, according to the present invention, since regions of high fiber density and regions of low fiber density do not exist in the surface side sheet, even when menstrual blood is absorbed, regions of high menstrual blood content and regions of low menstrual blood content will not exist in the surface side sheet and thus regions that are high and regions that are low in the shear strength with respect to the labial inner walls will not exist. Thus even in the highly wet state after absorption of menstrual blood, positional deviation and falling off of the interlabial pad can be prevented. The interlabial pad can thus be fitted securely between the labia even without the use of an adhesive agent.

Also, since the surface of the surface side sheet that is in contact with the labial inner walls is uniform in fiber density, the surface of the surface side sheet will be smooth. Since a large contact area with respect to the labial inner walls can thus be secured and the wet shear strength of the surface side sheet with respect to the labial inner walls will thus be high, positional deviation and falling off of the interlabial pad can be prevented further.

Here, that the surface is smooth means that there is no excess in terms of fluff on the surface due to fibers, blended amount of fibers of thick fiber diameter, blended amount of crimped synthetic fibers, open pores, etc. Specifically, the surface side sheet preferably has a surface roughness, as measured under the conditions of 50g/cm$^2$ (49N/m$^2$) load and surface measurement movement speed of 1 mm/sec using the "KES Surface Tester," manufactured by Kato-Tech Co., Ltd., of no more than 3.50μm.

Also, with regard to the fiber distribution of the surface side sheet, the standard deviation of the histogram measured using "Formation Tester FMT-MIII," manufactured by Nomura Shoji Co., Ltd., is preferably no more than 0.060 and more preferably 0.010 to 0.060.

As methods of making the fiber distribution dense, a method of increasing the humidity of the fibers so as to prevent the generation of static electricity among the fibers and a method of coating the fibers with a surface active agent to increase lubrication among fibers and thereby improving the fiber opening property may be cited.

(7) The above-described surface side sheet, wherein the fiber distribution of said surface side sheet is such that the standard deviation of a histogram as measured by "Formation Tester FMT-MIII," manufactured by Nomura Shoji Co., Ltd., is no less than 0.010 and no more than 0.060.

According to the present invention, the effects of (6) can be realized more definitely.

With a fiber distribution with which the standard deviation is greater than 0.060, the fibers will be poor in opening property and the fibers will therefore not be dispersed, causing formation of regions that are layered in lumps. Thus not only does the wet shear strength become non-uniform but the regions that are layered in lumps may damage the labial inner walls since these regions are high in rigidity. Meanwhile, with a fiber distribution with which the standard deviation is lower than 0.010, since the state of layering is extremely dense, there are hardly any density differences, so that the softness of the surface side sheet is lowered and the property of following the labial inner walls is poor.

(8) A surface side sheet for being used in an interlabial pad fitted between labia, comprising: a fiber aggregate wherein said fiber aggregate is a blend of two or more types of fibers which differ in fiber rigidity.

According to the present invention, by setting the water flow for entangling the fibers to a water pressure by which the fibers of lower fiber rigidity become entangled loosely with each other, the binding force of the fibers of higher rigidity can be lowered. The binding force among fibers can thereby be made looser in comparison to a case where the surface side sheet is formed of a single type of fiber. Thus even if, in the highly wet state after absorption of menstrual blood, an impact is applied to the surface side sheet due to movement of the wearer while the interlabial pad is fitted, this impact can be cushioned since the degree of freedom among the fibers is high. Since the applied impact will thus hardly act as a shear force on the interface of the surface side sheet and the labial inner walls, positional deviation and falling off of the interlabial pad can be prevented. The interlabial pad can thus be fitted securely between the labia even without the use of an adhesive agent.

Also, by suitably adjusting the blending ratio of the fibers that differ in fiber rigidity, the binding force among the fibers can be adjusted readily.

(9) The above-described surface side sheet, wherein said fibers which differ in fiber rigidity are a hydrophilic fiber of low fiber rigidity and a synthetic fiber of high fiber rigidity.

According to the present invention, the effects described for (8) can be realized definitely.

(10) An interlabial pad comprising: a surface side sheet facing labial inner walls, a back face side sheet facing garments, and an absorbent body sandwiched between said surface side sheet and said back face side sheet and absorbing body fluids, wherein said surface side sheet having a fiber aggregate, wherein a wet shear strength of said fiber aggregate with respect to labial inner walls is no less than 15cN/25mm and no more than 60cN/25mm where containing an artificial menstrual blood at a content of 800%, the artificial menstrual blood is a fluid composition comprising water, glycerin, sodium carboxymethylcellulose, sodium chloride, sodium bicarbonate, and a food dye preparation.

According to the present invention, even in a highly wet state after absorption of menstrual blood, positional deviation and falling off of the interlabial pad can be prevented. The interlabial pad can thus be fitted securely between the labia even without the use of an adhesive agent.

(11) The above-described interlabial pad, wherein an area of contact of said absorbent body with said surface side sheet is no more than 30% of an area of said absorbent body.

According to the present invention, since an impact from the absorbent body is less likely to be transmitted to the surface side sheet, which is loosened in the binding force among fibers, the positional deviation and falling off of the interlabial pad can be prevented further.

Here, at the surface side sheet side of the absorbent body, the percentage with respect to the surface area of the area onto which an adhesive agent is coated is preferably 0 to 30%, or the emboss percentage is preferably 0 to 30%.

In order to make an impact from the absorbent body be less likely to be transmitted to the surface side sheet, it is most preferable for the surface side sheet and the absorbent body not to be joined together. By making the parts of the surface side sheet and the back face side sheet that correspond to the peripheral parts of the absorbent body integral by means of an adhesive agent or an emboss process, a structure with which the surface side sheet and the absorbent body are not joined can be realized. With such a structure, since an adhesive agent is not coated, chemical irritation due to elution of an adhesive agent can be prevented. Since an emboss process is also not applied, regions of high fiber density that are due to an emboss process do not exist and thus physical irritation of the labial inner walls can also be lessened.

The pattern of coating of an adhesive agent is not restricted in particular and may be of an Ω-form, liquid-form, dot-form, etc. Also, in order to lessen irritation of the labial inner walls, a heat-sensitive adhesive agent is more preferable than a pressure-sensitive adhesive agent. Also, the amount of adhesive agent coated is preferably 1 to 20g/m$^2$.

Though a crimp pattern, dot pattern, lattice pattern, etc., may be considered for the emboss pattern, a dot pattern is preferable in view of softness.

(12) The above-described interlabial pad, wherein a total size in a lateral direction of said surface side sheet in a state where the interlabial pad is folded in two along a folding line provided along a longitudinal direction so that said surface side sheet is set outside is longer than that in a state where the interlabial pad is flat.

According to the present invention, the interlabial pad is arranged so that when it is folded in two along a folding line provided along the longitudinal direction so that the surface side sheet is set at the outer side, the total size in the lateral direction of the surface size sheet is longer than that in the planar state prior to folding. Thus even when the interlabial pad is folded in two, the surface side sheet will not be pressed by the absorbent body positioned inside the interlabial pad and the force that tends to return the interlabial pad to the state prior to folding, that is, the force that tends to open both ends of the interlabial pad can be restrained. Thus even when the interlabial pad is fitted between labia, the interlabial pad will not spread apart the ends of the labia and thus will not fall off.

Here, the planar state prior to folding of the interlabial pad refers to the state in which the interlabial pad is opened, and the total size in the lateral direction refers to the size from one edge to the other edge in the lateral direction of the interlabial pad.

The meaning of the total size in the lateral direction of the surface side sheet in the state where the abovementioned interlabial pad is folded in two being longer than the size in the lateral direction of the surface side sheet prior to folding shall now be described. That is, when the interlabial pad is folded in two along the longitudinal direction, the size in the lateral direction of the surface side sheet, which is positioned at the outermost side, is longer than the size in the lateral direction in the planar state prior to folding by approximately the circumferential length of a semicircle having the thickness of the interlabial pad as the radius.

The following methods may be cited as methods of making the total size in the lateral direction of the surface side sheet in the state in which the interlabial pad is folded in two longer than the total size in the lateral direction in the planar state. For example, there is a method of making the total size in the lateral direction of the surface side sheet greater than the total size in the lateral direction of the back face side sheet and performing joining to the surface side sheet in a state in which the surface side sheet is sagged in the lateral direction. There is also a method of folding the back face side sheet and the absorbent body in two and joining the surface side sheet and the back face side sheet in this state. There is also a method of making the surface side sheet readily extendable in the lateral direction and joining surface side sheet and back face side sheet in the planar state. Here, a crimping process or a slitting process may be applied along the lateral direction to the surface side sheet, or the surface side sheet itself may be provided with the characteristic of a maximum tensile elongation in the lateral direction of 100% or more. In view of the simplicity of the processing process, it is preferable for a surface side sheet formed by wet forming spun lacing to be subject to a tendering process and thereby made 100% or more in the maximum elongation in the lateral direction and then joined to the back face side sheet.

(13) The above-described interlabial pad, wherein the interlabial pad is for urine incontinence.

According to the present invention, the interlabial pad can be used as an absorbing product for urine incontinence. That is, since both the ostium vaginae, from which menstrual blood is discharged, and the urethral meatus, from which urine is discharged, are located between the labia, when the present invention's interlabial pad is used by being sandwiched between the labia, it can absorb urine. Since the urine can be absorbed between the labia and especially in the vicinity of the urethral meatus, the interlabial pad is effective for urine incontinence, especially, urine incontinence of a light degree.

(14) The above-described interlabial pad, wherein the interlabial pad is for vaginal discharge.

According to the present invention, the interlabial pad can be used for absorbing vaginal discharge. That is, by using the interlabial pad by sandwiching it between the labia, discharge (vaginal discharge) from the ostium vaginae, besides menstrual blood, can be absorbed. Since the vaginal discharge can be absorbed to alleviate the discomfort of a wearer, it is also effective for the interlabial pad to be fitted on at times besides during menstruation.

(15) A method of improving a adhesion of an interlabial pad to labial inner walls, said interlabial pad having a surface side sheet facing labial inner walls, a back face side sheet facing garments, and an absorbent body sandwiched between said surface side sheet and said back face side sheet and absorbing body fluids, comprising step of: forming said surface side sheet of a fiber aggregate wherein a binding force among fibers of said fiber aggregate is loose.

According to the present invention, the degree of freedom among the fibers that make up the surface side sheet is made high and even when an impact is applied to the surface side sheet due to movement of a wearer while the interlabial pad is fitted, this impact can be cushioned. Since the applied impact will thus hardly act as a shear force on the interface of the surface side sheet and the labial inner walls, positional deviation and falling off of the interlabial pad can be prevented.

(16) A method of lowering an area of coating of an adhesive agent for fitting an interlabial pad between labia, said interlabial pad comprising a surface side sheet facing labial inner walls, a back face side sheet facing garments, and an absorbent body sandwiched between said surface side sheet and said back face side sheet and absorbing body fluids, comprising step of: forming said surface side sheet of a fiber aggregate wherein a binding force among fibers of said fiber aggregate is loose.

According to the present invention, the degree of freedom among the fibers that make up the surface side sheet is made high and even when an impact is applied to the surface side sheet due to movement of a wearer while the interlabial pad is fitted, this impact can be cushioned. Since the applied impact will thus hardly act as a shear force on the interface of the surface side sheet and the labial inner walls, positional deviation and falling off of the interlabial pad can be prevented. Furthermore, since an adhesive agent is not coated, chemical irritation due to elution of an adhesive agent can be prevented and the manufacturing cost of the interlabial pad can be reduced.

**BRIEF DESCRIPTION OF THE DRAWINGS**

Fig. 1A is a perspective view showing an interlabial pad of an embodiment of the present invention.
Fig. 1B is a sectional view along line X-X of Fig. 1A.
Fig. 2A is a sectional view showing an interlabial pad of a first modification example of the present invention.
Fig. 2B is a sectional view showing an interlabial pad of a second modification example of the present invention.
Fig. 3 is a sectional view showing a state in which the interlabial pad of the abovementioned embodiment is folded in two.
Fig. 4 is an explanatory diagram for describing a method of testing the wet shear strength of the interlabial pad of the abovementioned embodiment.

**DESCRIPTION OF THE PREFERRED EMBODIMENT**

Though an embodiment of the present invention's interlabial pad shall now be described with reference to the drawings, the present invention is not limited to this embodiment.

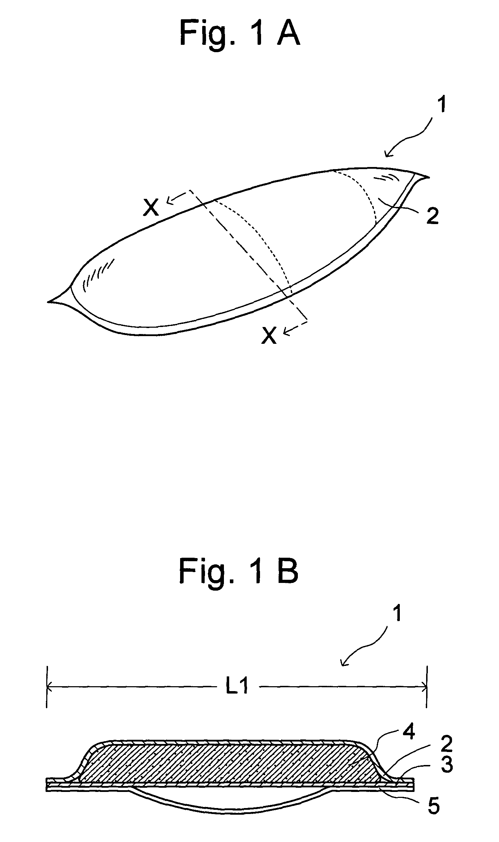

The basic arrangement of this embodiment's interlabial pad shall be described. Fig. 1A is a perspective view showing the interlabial pad of the present invention's embodiment and Fig. 1B is a sectional view along line X-X of Fig. 1A.

[Overall arrangement of the interlabial pad]

The present invention's interlabial pad 1 has a long, thin shape and, as shown in Figs. 1A and 1B, is equipped with a water-permeable surface side sheet 2, which faces labial inner walls, a water-permeable or non-water-permeable back face side sheet 3, which faces garments, and an absorbent body 4, which is enclosed by sheets 2 and 3 and absorbs body fluids. At the garment side of back face side sheet 3 is attached a mini-sheet piece 5, which spans the two side parts that extend along the longitudinal direction of interlabial pad 1.

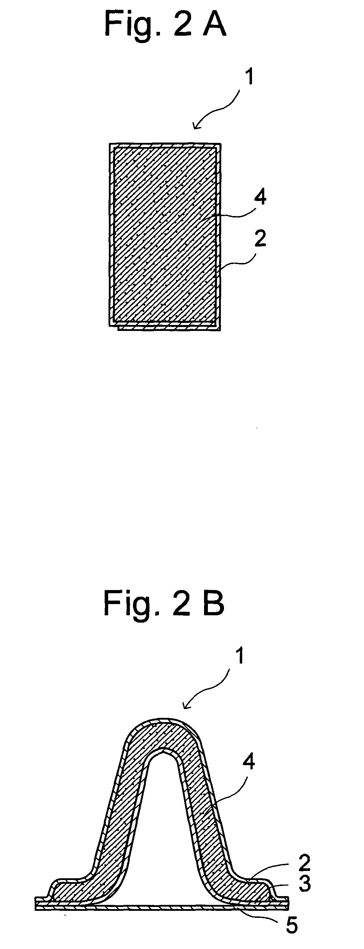

Though interlabial pad 1 of the present embodiment has a substantially oval shape in planar view, the shape is not restricted in particular with the present invention as long as the interlabial pad can be sandwiched and held between labia and the shape may be rectangular, gourd-shape, teardrop-shape, etc. Also, though the cross-sectional shape of interlabial pad 1 of this embodiment is a half-barrel shape, this shape is not restricted in particular with the present invention as long as the interlabial pad can be sandwiched and held between labia and may be rectangular, as shown in Fig. 2A, or a shape that is bent in a substantially V-like manner as shown in Fig. 2B.

[Component materials of the interlabial pad]

<Surface side sheet>

Surface side sheet 2 is a fiber aggregate formed by spun lacing a single type or a blend of predetermined amounts of several types of hydrophilic fibers, such as pulp, natural cotton, rayon, acetate, etc., to which a hydrophilic surface active agent is not attached.

Besides this, spun bonding, point bonding, through air method, needle punching, wet paper method, wet forming spun lacing, etc., may be cited as other methods of manufacturing the fiber aggregate.

Specifically, surface side sheet 2 is manufactured by the following procedures.

Rayon with a fineness of 1.1 to 4.4dtex and a fiber length of 7 to 51 mm, being of an amount that is 25 to 80% with respect to the total specific weight is layered at the labial inner wall side. Meanwhile, rayon with a fineness of 1.1 to 4.4dtex and a fiber length of 7 to 51 mm, being of an amount that is 45 to 14% with respect to the total specific weight, and PET with a fineness of 1.1 to 4.4dtex and a fiber length of 7 to 51mm, being of an amount that is 30 to 60% with respect to the total specific weight, are blended and layered at the absorbent body side. These two layers are layered on a conveyor that is fed at a speed of 70m/min so that the total specific weight becomes 20 to 60g/m$^2$, and the fibers are entangled with each other and made into a sheet by means of a water flow of a total water pressure of 120 to 140kg/cm$^2$ (converted value: 11.76 to 13.72MN/m$^2$) and a total water flow rate of 1920 to 2000cc/cm·min (converted value: 1.92 to 2.00l/cm·min) from three nozzles disposed in parallel (nozzle diameter: 92μm; nozzle pitch: 0.5mm). Thereafter this sheet is dried with air adjusted to 110°C and an air flow of 2m/sec and is adjusted in thickness to within a range of 0.13 to 0.50mm and in density to within a range of 0.040 to 0.400g/cm$^3$ (converted value: 40 to 400kg/m$^3$).

Here, the water pressure per nozzle for entangling the fibers with each other by means of water flow is preferably 50kg/cm$^2$ (converted value: 4.90N/m$^2$) or less. Also, the hydro-entanglement conditions are not limited to the above.

Though with the present embodiment, surface side sheet 2 is arranged as a layered body of two layers, the present invention is not limited thereto, and the surface side sheet may be arranged as a single layer. For example, rayon with a fineness of 1.1 to 4.4dtex, fiber length of 7 to 51 mm, and a specific weight of 20 to 60g/m$^2$ may be subject to entanglement of the fibers with each other and made into a sheet by hydro-entanglement (the water pressure in this case is preferably set to 50kg/cm$^2$ (converted value: 4.90MN/m$^2$) or less) and thereafter dried and adjusted in thickness to within a range of 0.13 to 0.50mm.

With this surface side sheet 2, since the binding force among fibers is made loose, even when an impact is applied to the surface side sheet by movement of a wearer, since the degree of freedom of the fibers is high, the impact can be cushioned by air gaps between fibers and the wet shear strength of the surface side sheet as a whole can be maintained.

<Back face side sheet>

Back face side sheet 3 prevents menstrual blood held by the absorbent body from leaking out of interlabial pad 1. This back face side sheet may be arranged as a moisture-permeable material. By doing so, mustiness during fitting of the interlabial pad can be lessened and the discomfort during fitting of the interlabial pad can be resolved.

As back face side sheet 3, for example, an air-permeable film, which is obtained by applying an elongation process to a sheet-like film, made by forming a synthetic resin into a film, and an inorganic filler, or a laminate, which is prepared as a composite of paper, a nonwoven fabric, and a film, or an air-permeable, liquid-blocking sheet, having capillaries with a pore diameter of 0.1 to 0.6mm, etc., at an amount of 10 to 30%, may be used.

Furthermore, as the back face side sheet, a film with a specific weight of 15 to 30g/m$^2$ and mainly comprising a low density polyethylene (LDPE) resin with a density of 0.900 to 0.925g/cm$^3$ (converted value: 900 to 925kg/m$^3$) may be used. By doing so, the back face side sheet can be provided with softness and the feel of fitting can be improved.

<Absorbent body>

Absorbent body 4 absorbs and holds fluids (body fluids).

As absorbent body 4, a material that is bulky, is not deformed readily, and is low in chemical irritability is preferable. Specifically as absorbent body 4, a pulp, chemical pulp, rayon, acetate, natural cotton, super absorbent polymer, super absorbent polymer fibers, or synthetic fibers may be used solitarily or in blended form.

Specifically, the absorbent body is manufactured by the following procedures.

Rayon or acetate, selected from among those in a range of 1.1 to 4.4dtex, is layered at a blending percentage of 60 to 90% with 10 to 40% of super absorbent polymer fibers. The layered fibers are then entangled by needling and formed into a sheet to prepare a nonwoven fabric sheet with a specific weight of 150 to 500g/m$^2$ and a bulk of 2 to 5mm.

The above-described absorbent body 4 is overlapped, folded, etc., and thereby adjusted suitably in bulk in the process of incorporation in the interlabial pad.

<Mini-sheet piece>

For mini-sheet piece 5, though the same material as that of surface side sheet 2 or back face side sheet 5 described above may be used, a material that is stretchable at least in the lateral direction is preferably used.

By forming mini-sheet piece 5 from a material with stretchability, even when a fingertip of a wearer is larger in size than a finger insertion opening (a gap between mini-sheet piece 5 and back face side sheet 3), the fingertip of the wearer can be inserted definitely into the finger insertion opening since the mini-sheet piece stretches in this case.

Examples of materials with stretchability include films, open-pore foam films, nets, etc., formed of styrene-butadiene-styrene block-copolymer (SBS), styrene-isoprene-styrene block copolymer (SIS), urethane or other synthetic rubber or an amorphous olefin type resin selected from among those with a density of 0.88 to 0.900g/cm$^3$ (converted value: 880 to 900kg/m$^3$) as the raw material. Examples besides the above include woven fabrics, fabrics in which spun filaments formed of synthetic rubber as the raw material are woven into a woven fabric, spun bonded nonwoven fabrics having synthetic rubber as the main component, melt-blown nonwoven fabrics, foam sheets, etc.

As an abovementioned open-pore foam film, a film formed of SEBS, which is adjusted in thickness of 15 to 40 microns and arranged to have a pore area of 0.28 to 1.77mm$^2$ (converted value: 0.28 to $1.77\times10^{-6}$m$^2$) and a porosity in a range of 40 to 70%, is favorable in terms of providing a soft texture upon fitting.

Examples of the abovementioned nonwoven fabric include those having heat-shrinkable composite synthetic fibers of PE/PP, PE/PET, PP/PP, etc., with a high melting point core component and a low melting point sheath component, as the raw material and arranged as spun laced nonwoven fabrics, with which the fibers are entangled by water flow pressure, shrink-type nonwoven fabrics, with which the shrinkage of the fibers has been promoted by re-treatment by hot air, so-called stretchable spun bonded fabrics, with which continuous length fibers are formed into a sheet by heat sealing and thereafter forcibly tentered in the longitudinal direction, or laminates of such fabrics, etc.

Among shrink-type nonwoven fabrics, those having, as the raw material, heat-shrinkable composite synthetic fibers of PE/PP, PE/PET, PP/PP, etc., which have a high melting point core component and a low melting point sheath component and are 2.2 to 6.6dtex in thickness, 38 to 51 mm in length, and adjusted in specific weight to within a range of 20 to 60g/m$^2$, are favorable since such fabrics are soft and rich in draping property.

In a case where non-shrinkable material is to be used upon being provided with shrinkability, examples of nonwoven fabrics that can be used include those having heat-shrinkable composite synthetic fibers of PE/PP, PE/PET, PP/PP, etc., with a high melting point core component and a low melting point sheath component, as the raw material and are arranged as through-air nonwoven fabrics, which have been treated with hot air and are rich in bulkiness, spun laced nonwoven fabrics, with which the fibers are entangled by water flow pressure, spun bonded nonwoven fabrics, with which continuous fibers are layered and formed into a sheet, needle punched nonwoven fabrics, with which the fibers are entangled with each other by a needle, or SMS nonwoven fabrics, with which spun bonded fibers and melt blown fibers are layered in multiple layers and formed into a sheet. Besides these, an open pore foam film, a film having PE resin as the main component, etc., may be used solitarily or a composite material of the above may be used.

Also, an abovementioned non-shrinkable material may be provided with stretchability by a corrugating process in which the material is fitted between male and female dies and shapes are embossed by heat, temperature, and pressure. A through-air nonwoven fabric, having composite synthetic fibers adjusted to a thickness of 2.2 to 4.4dtex and a specific weight of 20 to 60g/m$^2$ as the main component, and which is subject to a corrugating process so as to be stretchable in the lateral direction, may be cited as a specific example.

With a corrugated fabric, the stretchability should be at least 10%, more preferably the stretchability is 20 to 50%, and even more preferably, the load at 30% stretching is 0.01 to 0.05N/25mm (test conditions: speed of 100mm/min (converted value: 6m/h) and chuck interval of 100mm with a Tensilon Tensile Tester).

As other methods of providing stretchability, the methods of providing the mini-sheet piece with slits, cutting the mini-sheet piece in a circle, etc., may also be used.

[Component materials of an interlabial pad provided with biodegradability, water dispersibility, and water solubility]

The present invention's interlabial pad is preferably formed of a biodegradable material and/or a water dispersible material and/or a water soluble material. Such an interlabial pad can be dropped in and wash down a toilet as it is after absorbing menstrual blood, etc., thus enabling simple and sanitary disposal of the interlabial pad as well as reduction of toilet waste.

Here, "biodegradability" refers to the property of decomposition into a gas, such as carbon dioxide, methane, etc., water, and biomass under the presence of microorganisms, such as actinomycetes and other microbes and under anaerobic or aerobic conditions. "Water dispersibility" is the same as water degradability and refers to the property of not changing in shape with a predetermined amount of water (menstrual blood) but being readily dispersed by a large amount of water into small fragments with which the fibers will not clog toilet piping. "Water solubility" refers to the property of not changing in shape with a predetermined amount of water (menstrual blood) but dissolving in a large amount of water.

<Surface side sheet>

As the surface side sheet, in addition to pulp, cotton, rayon, and acetate, a biodegradable resin, such as polylactic acid, polybutylene succinate, etc., may be used.

<Absorbent body>

As the absorbent body, a nonwoven fabric obtained by needling may be used. In view of the biodegradability, etc., of a super absorbent polymer material, carboxymethylcellulose fibers are preferable.

<Back face side sheet>

As the back face side sheet, laminate paper, with which a PVA film, a film sheet, with which one side or both sides of a PVA film is subject to water repellent treatment by silicone, etc., a PVA film having silicone mixed in, a starch film, or a film having a biodegradable resin, prepared by hydrolysis of polylactic acid or polybutylene succinate, etc., as the raw material, is laminated with tissue paper, etc., may be used. Also, an inorganic pigment may be mixed in a range of 0.1 to 5% for coloration as necessary.

In view of maintaining leakage prevention under excessive wetting and not applying an excessive impact on a septic tank, etc., laminated paper, with which a film, having polylactic acid as the raw material, is laminated to a thickness of 10 to 20μm onto tissue paper with a specific weight of 15 to 20g/m$^2$ and with which the percentage of the area adhered together in the lamination process is 5 to 40%, is favorable.

<Mini-sheet piece>

As the mini-sheet piece, a film, a spun bonded nonwoven fabric, or a melt blown nonwoven fabric, etc., having polylactic acid, polybutylene succinate, or other biodegradable material as the raw material, a film or nonwoven fabric, etc., having PVA, CMC, or other water soluble material as the raw material, a water-dispersible tissue or spun laced nonwoven fabric, etc., having cellulose fibers or regenerated cellulose fibers, etc., as the main component, etc., may be used.

Among these, a spun bonded nonwoven fabric or melt blown nonwoven fabric, which is mainly formed of biodegradable material and is a sheet that has been adjusted to a thickness in a range of 0.1 to 3.3dtex and a specific weight in a range of 15 to 40g/m$^2$ and has been subject to the above-described mechanical corrugating process, is favorable.

[Arrangement of the interlabial pad]

Interlabial pad 1 is, for example, folded in two and fitted between labia, and here, if the total size L in the lateral direction of surface side sheet 2 in the folded state is equal to the size L1 in the lateral direction of surface side sheet in the non-folded state or is slightly longer than the size L1, the part of surface side sheet 2 at the body side, which is the outermost surface, protrudes due to the thickness of absorbent body 4. As a result, even when interlabial pad 1 is fitted between labia in a state in which it is folded in two, a force that tends to open the end edges of the folded interlabial pad 1 acts to open up the edges of the labia so that interlabial pad 1 may fall off.

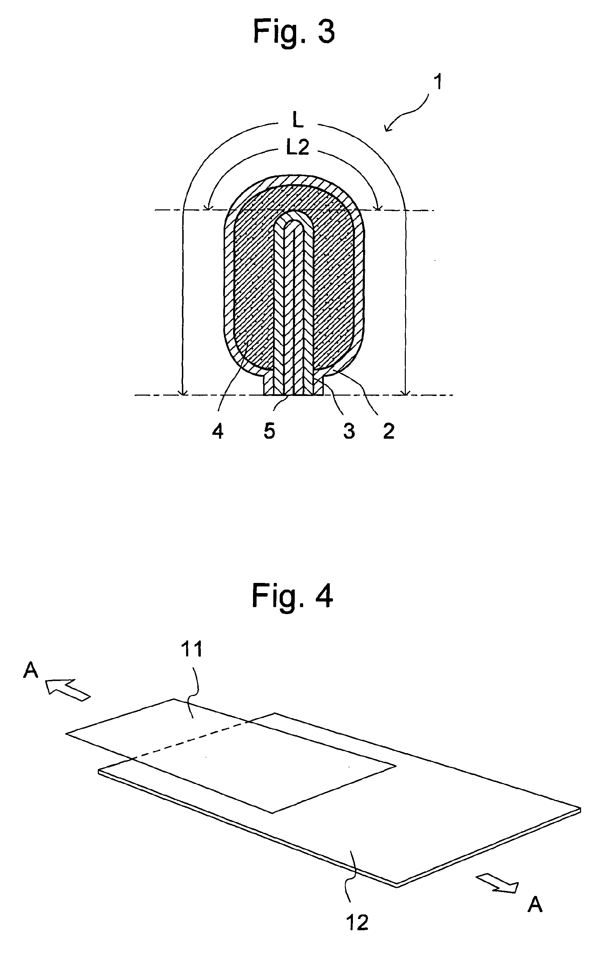

Thus as shown in Fig. 3, interlabial pad 1 is folded in two so that back face side sheet 3 contacts itself along the central line in the longitudinal direction. Here, the total size L in the lateral direction of surface side sheet 2 in the state in which it is folded in two will at least be the sum of the apparent size L1 in the lateral direction in the planar state prior to folding (see Fig. 1B) and the length L2 (stretch length), equivalent to the circumferential length of a semicircle having the thickness of interlabial pad 1 in the planar, opened state as the radius. Surface side sheet 2 is thereby prevented from being pressed by absorbent member 4 and the force that tends to return interlabial pad 1 to the state prior to folding, that is, the force that tends to open up the edges at both sides can be restrained. Thus even if interlabial pad 1 is fitted upon being folded in two, there is no danger of interlabial pad 1 falling off.

The following methods may be cited as methods of making the total size in the lateral direction of surface side sheet 2 longer than the total size in the lateral direction in the planar state. For example, there is a method of making the total size in the lateral direction of the surface side sheet greater than the total size in the lateral direction of the back face side sheet and performing joining to back face side sheet 3 in a state in which surface side sheet 2 is sagged in the lateral direction. There is also a method of folding back face side sheet 3 and absorbent body 4 in two and joining surface side sheet 2 to back face side sheet 3 in this state. There is also a method of making surface side sheet 2 readily stretchable in the lateral direction and joining surface side sheet 2 and back face side sheet 3 in the planar state. Here, a crimping process or a slitting process along the lateral direction may be applied to surface side sheet 2, or surface side sheet 2 may be provided with the characteristic of a maximum tensile elongation in the lateral direction of 100% or more.

Though interlabial pad 1 of the present embodiment has a mini-sheet piece 5 attached to the garment side of back face side sheet 3 in a manner so as to span the two side parts that extend along the longitudinal direction of interlabial pad 1, the present invention is not limited to this shape and, for example, in place of providing mini-sheet piece 5, a protrusion may be provided on the surface at the opposite side (garment side) of the body side and this protrusion may be held with two fingers to fit on the interlabial pad. Furthermore, a mini sheet or a protrusion does not have to be provided at all.

[Examples]

<Example 1>

A surface side sheet is prepared by the following procedures. First, rayon, with a fineness of 1.4dtex and a fiber length of 44mm, is layered on a conveyor fed at a speed of 70m/min so that the specific weight becomes 33g/m$^2$. The layered rayon is then subject to entanglement of the fibers with each other and made into a sheet by means of a water flow of a total water pressure of 120kg/cm$^2$ (converted value: 11.76MN/m$^2$) and a total water flow rate of 1920cc/cm·min (converted value: 1.92l/cm·min) from three nozzles disposed in parallel (nozzle diameter: 92μm; nozzle pitch: 0.5mm). The sheet is then dried with air set to 110°C and an air flow of 2m/sec, and is thereby made into a single-layer spun laced nonwoven fabric that is adjusted to a thickness of 0.31mm and a density of 0.110g/cm$^3$ (converted value: 110kg/m$^3$).

<Example 2>

A surface side sheet is prepared by the following procedures. First, for the labial inner wall side, rayon with a fineness of 1.4dtex and a fiber length of 44mm, being of an amount that is 50% with respect to the total specific weight, is layered. Meanwhile, for the absorbent body side, rayon with a fineness of 1.4dtex and a fiber length of 44mm, being of an amount that is 35% with respect to the total specific weight, and PET with a fineness of 1.3dtex and a fiber length of 38mm being of an amount that is 15% with respect to the total specific weight, are blended and layered. These two layers are layered on a conveyor that is fed at a speed of 70m/min so that the total specific weight becomes 33g/m$^2$, and the fibers are entangled with each other and made into a sheet by means of a water flow of a total water pressure of 140kg/cm$^2$ (converted value: 13.72MN/m$^2$) and a total water flow rate of 2000cc/cm·min (converted value: 2.00l/cm·min) from three nozzles disposed in parallel (nozzle diameter: 92μm; nozzle pitch: 0.5mm). Thereafter, this sheet is dried with air set to 110°C and an air flow of 2m/sec, and is thereby made into a two-layer spun laced nonwoven fabric that is adjusted to a thickness of 0.350mm and a density of 0.093g/cm$^3$ (converted value: 93kg/m$^3$).

<Comparative Example 1>

A surface side sheet is prepared by the following procedures. First, rayon, with a fineness of 1.4dtex and a fiber length of 44mm, is layered on a conveyor fed at a speed of 70m/min so that the specific weight becomes 33g/m$^2$. The layered rayon is then subject to entanglement of the fibers with each other and made into a sheet by means of a water flow of a total water pressure of 180kg/cm$^2$ (converted value: 17.64MN/m$^2$) and a total water flow rate of 2220cc/cm·min (converted value: 2.22l/cm·min) from three nozzles disposed in parallel (nozzle diameter: 92μm; nozzle pitch: 0.5mm). The sheet is then dried with air set to 110°C and an air flow of 2m/sec, and is thereby made into a single-layer spun laced nonwoven fabric that is adjusted to a thickness of 0.27mm and a density of 0.123g/cm$^3$ (converted value: 123kg/m$^3$).

<Comparative Example 2>

A surface side sheet is prepared by the following procedures. First, rayon with a fineness of 1.4dtex and a fiber length of 44mm, being of an amount that is 70% with respect to the total specific weight, and PET with a fineness of 1.3dtex and a fiber length of 38mm, being of an amount that is 30% with respect to the total specific weight, are blended and these fibers are layered on a conveyor that is fed at a speed of 70m/min so that the total specific weight becomes 33g/m$^2$, and the fibers are entangled with each other and made into a sheet by means of a water flow of a total water pressure of 180kg/cm$^2$ (converted value: 17.64MN/m$^2$) and a total water flow rate of 2220cc/cm·min (converted value: 2.22l/cm·min) from three nozzles disposed in parallel (nozzle diameter: 92μm; nozzle pitch: 0.5mm). Thereafter, this sheet is dried with air set to 110°C and an air flow of 2m/sec, and is thereby made into a single-layer spun laced nonwoven fabric that is adjusted to a thickness of 0.29mm and a density of 0.113g/cm$^3$ (converted value: 113kg/m$^3$).

<Comparative Example 3>

A surface side sheet is cut apart from an interlabial pad (trade name: "Envive") manufactured by P & G Inc. and this surface side sheet is evaluated as Comparative Example 3 in the same manner as the other samples.

Analysis by micro-infrared analysis, etc., showed this surface side sheet to be a single-layer chemical binder nonwoven fabric with the fiber portion being cellulose-type regenerated fibers with a fineness of approximately 1.4dtex and a fiber length of approximately 44mm. The main component of the binder portion of this binder nonwoven fabric is a mixture of a paraffin type wax and an aliphatic polyether type wax and the component ratio of the fiber portion and the binder portion is 60:40.

<Comparative Example 4>

A surface side sheet is prepared by the following procedures. First, after coating a hydrophilic lubricant at an amount of 0.4% to heat-shrinkable composite synthetic fibers of PE/PP, having a core component of high melting point, a sheath component of low melting point, a fineness of 4.4dtex, and a fiber length of 51 mm, the fibers are layered on a conveyor that is fed at a speed of 90m/min so that the total specific weight becomes $20g/m^2$. Thereafter, the fibers are fused with each other by air set to $140°C$ and an air flow of 2m/sec, and are thereby made into a single-layer through air nonwoven fabric that is adjusted to a thickness of 0.71mm and a density of $0.028g/cm^3$ (converted value: $28kg/m^3$).

The characteristics (density, tensile strength, water absorption, surface characteristics, etc.) of the surface side sheet of each of the above-described Examples and Comparative Examples and the fall-off percentages and adhesion to labial inner walls (shear strength) of interlabial pads using the respective surface side sheets are shown in Table 1.

[Table 1]

| | | Example 1 | Example 2 | Comparative Example 1 | Comparative Example 2 | Comparative Example 3 | Comparative Example 4 |
|---|---|---|---|---|---|---|---|
| Manufacturing method<br>Arrangement | | Spun lacing<br>Single layer | Spun lacing<br>Two layers | Spun lacing<br>Single layer | Spun lacing<br>Single layer<br>(blended fabric) | Chemical binder<br>Single layer<br>(blended fabric) | Through air<br>Single layer |
| WJ nozzle (diameter: 92μm, pitch: 0.5mm)<br>Pressure at each nozzle          kg/cm$^2$<br>Water flow rate at each nozzle     cc/cm·min<br>Total nozzle water flow rate       cc/cm·min | | 3 nozzles<br>40+40+40<br>640+60+640<br>1920 | 3 nozzles<br>40+50+50<br>640+680+680<br>2000 | 3 nozzles<br>60+60+60<br>740+740+740<br>2220 | 3 nozzles<br>60+60+60<br>740+740+740<br>2220 | --- | --- |
| Composition | Upper layer | Rayon: 100%<br>(1.4dt×44) | Rayon: 50%<br>(1.4dt×44) | Rayon: 100%<br>(1.4dt×44) | Rayon: 70%<br>(1.4dt×44) | Rayon: 60%<br>(1.4dt×44) | PE/PP:100%<br>(1.4dt×44) |
| | Lower layer | | Rayon: 35%<br>(1.4dt×44)<br>PET: 15%<br>(1.3dt×38) | | PET: 15%<br>(1.3dt×38) | Paraffin wax: 40% | Hydrophilic<br>lubricant:<br>minute amount |
| Specific weight          g/m$^2$ | | 33.0 | 33.0 | 33.1 | 32.7 | 23.0 | 20.0 |
| Thickness | In dry state (under load of 0.5g/cm$^2$)          mm | 0.30 | 0.35 | 0.27 | 0.29 | 0.11 | 0.71 |
| | In wet state (at artificial menstrual blood content of 800% and under load of 0.5g/cm$^2$)          mm | 0.24 | 0.31 | 0.21 | 0.26 | 0.10 | 0.40 |
| Thickness ratio (wet thickness / dry thickness)% | | 80 | 89 | 78 | 90 | 91 | 56 |
| Wet shear strength (longitudinal direction) | At artificial menstrual blood content of 300%          cN/25mm | 35 | 37 | 33 | 35 | 41 | 3 |
| | At artificial menstrual blood content of 800%          cN/25mm | 22 | 29 | 14 | 11 | 8 | 4 |
| Percentage of fall-off during walking in fitting test  % | | 0 | 0 | 13 | 20 | 40 | 40 |
| Surface characteristics | SMD          μm | 2.79 | 2.82 | 2.56 | 2.56 | 2.34 | 4.35 |
| Tensile strength in dry state (longitudinal direction) | 5% elongation          cN/25mm | 385 | 563 | 645 | 613 | 818 | 358 |
| Tensile strength at artificial blood content of 800% (longitudinal direction) | 5% elongation          cN/25mm | 182 | 135 | 275 | 291 | 10 | 220 |
| Klemm absorption | Longitudinal direction          mm | 30 | 25 | 31 | 19 | 12 | 1 |
| | Lateral direction          mm | 22 | 22 | 21 | 17 | 12 | 1 |

Conversion formulae:     1)    Pressure 1kg/cm$^2$ = 9.8×10$^4$N/m$^2$, 1g/cm$^2$ = 98N/m$^2$
2)    Nozzle flow rate    1cc/cm$^{-3}$l·min = 10-31/cm·min

EP 1 547 559 A1

[Test methods]

The test methods for evaluating the respective characteristics indicated in Table 1 shall now be described.

<Check of fall-off during walking by a fitting test>

A test piece is prepared by adhering a surface side sheet to the surface of a rectangular parallelepiped piece of 80mm length, 5mm width, and 20mm height formed of foamed polyethylene. Artificial menstrual blood is sprayed in a mist-like form onto the surface of this test piece to put the test piece in a wet state in which the artificial menstrual blood content in the test piece is 800%. Next, this test piece in the wet state is fitted between the labia of a wearer, the wearer is made to walk at a speed of 5km/h for 30 seconds on a walking machine (EX-W8000 Walker 8000, manufactured by Alinco Inc.), and whether or not the test piece fell off from between the labia is checked visually during these 30 seconds.
With each wearer, a test piece was fitted five times and the test was conducted with three wearers to determine the percentage of test pieces that fell off.

<Wet shear strength>

As shown in Fig. 4, from each surface side sheet, 10 pieces of nonwoven fabric of a width of 25mm and a length of 40mm are sampled randomly in the longitudinal and lateral directions as test pieces 11. To prepare test pieces with a predetermined content of artificial menstrual blood, artificial menstrual blood is sprayed in a mist-like form from the labial inner wall side of each test piece 11. The entire test piece 11 is then covered with a liquid-impermeable material and left for approximately 1 minute under an environment of 20°C and 60% humidity so that the artificial menstrual blood will spread throughout the entire test piece 11. With each test piece thus prepared, the side onto which the artificial menstrual blood was sprayed is adhered onto a stainless steel plate 12 with a width of 50mm, length of 60mm, and thickness of 2mm (see Fig. 4) and left pressurized at 10kg/cm$^2$ (converted value: 0.98MN/m$^2$) for 30 seconds. Thereafter, each test piece is pulled in the direction of arrow A in Fig. 4 in a constant extension rate tensile tester (AGS-1 kNG, manufactured by Shimadzu Corp.) to measure the maximum shear load value. The test conditions are set to an adhesion length of 30mm, a stress rate of 100mm/min (converted value: 6m/h), and a chuck interval of 40mm. For stainless steel plate 12, a SUS 304 steel plate as defined in JIS G4305 is surface finished in accordance with No. 4 as defined in JIS G 4305 and then subject to HL (hairline) finishing by a #200 polishing agent.

<Klemm absorption>

10 pieces of nonwoven fabric of a width of 25mm and a length of 100mm are sampled randomly in the longitudinal and lateral directions as test pieces from each surface side sheet. Each test piece is set so that the tip of the test piece is immersed to a depth of 5mm in artificial menstrual blood placed to a depth of 10mm or more in a plastic container and then left for 10 minutes. The absorption distance by which the fluid rose along the test piece is then measured.

<Tensile strength of the nonwoven fabric of a surface side sheet>

10 pieces of nonwoven fabric of a width of 100mm and a length of 100mm are sampled randomly in the longitudinal and lateral directions as test pieces from each surface side sheet. Each test piece is set in an automated tension/shear tester (KES-FB1-AUTO-A, manufactured by Kato-Tech Co., Ltd.) and the tensile strength at an elongation of 5% is measured.

<Surface characteristics of a surface side sheet>

10 pieces of nonwoven fabric of a width of 100mm and a length of 100mm are sampled randomly in the longitudinal and lateral directions as test pieces from each surface side sheet. Each test piece is set in an automated surface tester (KES-FB4-AUTO-A, manufactured by Kato-Tech Co., Ltd.) and the vertical thickness variation of the surface of the nonwoven fabric is measured.

<Relationship of the wet shear strength of a surface side sheet to the falling off of an interlabial pad>

The relationship of the wet shear strength of a surface side sheet to the falling off of an interlabial pad is checked by using surface side sheets that differ in wet shear strength and evaluating the "percentage of fall-off during walking by a fitting test" with three wearers.

[Test results]

With Example 1, the surface side sheet had a density of 0.110g/cm$^3$ (converted value: 110kg/m$^3$) and a tensile strength in a state of 5% elongation in the longitudinal direction of 385cN/25mm. The wet shear strength in a state of containing artificial menstrual blood at a content of 800% was 22cN/25mm and falling off of the interlabial pad did not occur.

With Example 2, the surface side sheet had a density of 0.094g/cm$^3$ (converted value: 94kg/m$^3$) and a tensile strength in a state of 5% elongation in the longitudinal direction of 563cN/25mm. The wet shear strength in a state of containing artificial menstrual blood at a content of 800% was 29cN/25mm and falling off of the interlabial pad did not occur.

Also, the ratio of the bulk in the wet state to the bulk in the dry state was 89% and thus a high bulk was maintained in comparison to a single-layer item. It is considered that a high bulk could be maintained even in the wet state since hydrophilic fibers and synthetic resin fibers are blended and layered at the absorbent body side.

With Comparative Example 1, the surface side sheet had a density of 0.123g/cm$^3$ (converted value: 123kg/m$^3$) and a high tensile strength in a state of 5% elongation in the longitudinal direction of 645cN/25mm. However, the wet shear strength in a state of containing artificial menstrual blood at a content of 800% was of a low value of 14cN/25mm and falling off of the interlabial pad occurred. This is considered to be due to the density being high, the binding force among the fibers being high, and thus the fibers being low in the degree of freedom.

With Comparative Example 2, the surface side sheet had a density of 0.113g/cm$^3$ (converted value: 113kg/m$^3$) and a high tensile strength in a state of 5% elongation in the longitudinal direction of 613cN/25mm. However, the wet shear strength in a state of containing artificial menstrual blood at a content of 800% was of a low value of 11 cN/25mm and falling off of the interlabial pad occurred.

Here, since fibers that differ in wetting property are blended to form the surface side sheet, regions that differ in absorption exist and the wet shear strength of the surface side sheet with respect to the labial inner walls is non-uniform. It is thus considered that the above results are due to regions of low wet shear strength separating readily from the labial inner walls upon movement of the wearer.

With Comparative Example 3, the surface side sheet had a density of 0.209g/cm$^3$ (converted value: 209kg/m$^3$) and a high tensile strength in a state of 5% elongation in the longitudinal direction of 818cN/25mm. Also, though the wet shear strength in a state of containing artificial menstrual blood at a content of 300% was of a high value of 41 cN/25mm, the wet shear strength in a state of containing artificial menstrual blood at a content of 800% was of a low value of 8cN/25mm. Furthermore, the tensile strength in a state of containing artificial menstrual blood at a content of 800% and elongating by 5% in the longitudinal direction was of a low value of 10cN/25mm and the percentage of fall-off of the interlabial pad was also high.

Here, in a state of low menstrual blood content, since the paraffin wax that is the chemical binder softens and becomes a highly viscous gel, the surface side sheet adheres closely to the labial inner walls and the wet shear strength is high. However in a state of high menstrual blood content, it is considered that the paraffin wax softens excessively and cannot maintain the form of a sheet.

With Comparative Example 4, the surface side sheet had a low wet shear strength in a state of containing artificial menstrual blood at a content of 300% of 3cN/25mm and a low wet shear strength in a state of containing artificial menstrual blood at a content of 800% of 4cN/25mm and was also high in the percentage of fall-off of the interlabial pad.

Here, since the sheet is formed by the fusion of fibers with each other, the density becomes low. As a result, the surface state is rough (the surface characteristic SMD is 4.35) and it is thus considered that the area of contact with the labial inner walls is low. Furthermore, though a hydrophilic lubricant is coated onto the surfaces of the fibers, since the surface side sheet is formed of 100% synthetic fibers, the Klemm absorption is only 1 mm for both the longitudinal direction and the lateral direction, the affinity with menstrual blood is low, and since it is thus difficult for even a small amount of menstrual blood to be absorbed by the surface side sheet, the wet shear strength of the surface side sheet with respect to the labial inner walls is lowered.

Also since the percentage of fall-off decreases as the wet shear strength increases, the relationship between the wet shear strength of the surface side sheet and the falling off of the interlabial pad was confirmed.

That is, within an artificial menstrual blood content range of 300 to 800%, the possibility that an interlabial pad will fall off will be low if the wet shear strength in the longitudinal direction of the labial inner wall side of a surface side sheet is no less than 15cN/25mm.

The present invention's surface side sheet and interlabial pad provides the following effects.

The surface side sheet is formed of a fiber aggregate having a wet shear strength with respect to labial inner walls of no less than 15cN/25mm and no more than 60cN/25mm in a state in which the content of artificial menstrual blood is 800%. Thus even when the shape of the labia changes due to movement of a wearer in a highly wet state after absorption of menstrual blood, since the shear force that arises between the labial inner walls and the interlabial pad can be resisted, positional deviation and falling off of the interlabial pad can be prevented. The interlabial pad can thus be fitted securely between the labia even without using an adhesive agent.

Also, the surface side sheet is formed of a fiber aggregate having a tensile strength of no more than 600cN/25mm in a state where the content of artificial menstrual blood is 300% and the surface side sheet is elongated by 5% in the longitudinal direction and exhibits a tensile strength of no less than 100cN/25mm in a state where the content of artificial menstrual blood is 800% and the surface side sheet is elongated by 5% in the longitudinal direction. Thus even when the surface side sheet absorbs blood and is put in a highly wet state, the binding force among the fibers that make up the surface side sheet will be loose and the degree of freedom among the respective fibers will be high. Thus even when an impact is applied to the surface side sheet due to movement of a wearer while the interlabial pad is fitted, this impact can be cushioned. Since the applied impact will thus hardly act as a shear force on the interface of the surface side sheet and the labial inner walls, positional deviation and falling off of the interlabial pad can be prevented. The interlabial pad can thus be fitted securely between the labia even without the use of an adhesive agent.

Also, the surface side sheet is formed of a fiber aggregate, having a dense fiber distribution with which the state of contact with labial inner walls will not be non-uniform. Since regions of high fiber density and regions of low fiber density will therefore not exist in the surface side sheet, even when menstrual blood is absorbed, regions of high menstrual blood content and regions of low menstrual blood content will not occur in the surface side sheet and thus regions that are high and regions that are low in the shear strength with respect to the labial inner walls will not exist. Thus even in the highly wet state after absorption of menstrual blood, positional deviation and falling off of the interlabial pad can be prevented. The interlabial pad can thus be fitted securely between the labia even without the use of an adhesive agent.

Also, the surface side sheet is formed of a fiber aggregate in which two or more types of fibers that differ in fiber rigidity are blended and entangled. Thus by setting the water flow for entangling the fibers to a water pressure by which the fibers of lower fiber rigidity become entangled loosely with each other, the binding force of the fibers of higher rigidity can be lowered. The binding force among fibers can thereby be made looser in comparison to a case where the surface side sheet is formed of a single type of fiber. Thus even if, in the highly wet state after absorption of menstrual blood, an impact is applied to the surface side sheet due to movement of the wearer while the interlabial pad is fitted, this impact can be cushioned since the degree of freedom among the respective fibers is high. Since the applied impact will thus hardly act as a shear force on the interface of the surface side sheet and the labial inner walls, positional deviation and falling off of the interlabial pad can be prevented. The interlabial pad can thus be fitted securely between the labia even without the use of an adhesive agent.

**Claims**

1. A surface side sheet for being used in an interlabial pad fitted between labia, comprising:

   a fiber aggregate, wherein a wet shear strength of said fiber aggregate with respect to labial inner walls is no less than 15cN/25mm and no more than 60cN/25mm where containing an artificial menstrual blood at a content of 800%, the artificial menstrual blood is a fluid composition comprising water, glycerin, sodium carboxymethylcellulose, sodium chloride, sodium bicarbonate, and a food dye preparation.

2. The surface side sheet according to claim 1, wherein a bulk of said fiber aggregate in a wet state is no less than 80% of a bulk in a dry state.

3. The surface side sheet according to claim 2, wherein said fiber aggregate comprises:

   a surface layer formed of a hydrophilic fiber and
   a back layer formed of a hydrophilic fiber and a synthetic resin fiber.

4. A surface side sheet for being used in an interlabial pad fitted between labia, comprising:

   a fiber aggregate having a tensile strength of no more than 600cN/25mm in a state where the surface side sheet contains an artificial menstrual blood at a content of 300% and is elongated by 5% in a longitudinal direction and having a tensile strength of no less than 100cN/25mm in a state where the surface side sheet contains said artificial menstrual blood at a content of 800% and is elongated by 5% in a longitudinal direction, the artificial menstrual blood is a fluid composition comprising water, glycerin, sodium carboxymethylcellulose, sodium chloride, sodium bicarbonate, and a food dye preparation.

5. The surface side sheet according to any one of claims 1 to 4, wherein a Klemm absorption of said artificial menstrual blood for 10 minutes is no less than 10mm.

6. A surface side sheet for being used in an interlabial pad fitted between labia, comprising:

   a fiber aggregate having a dense fiber distribution so as not to contact with labial inner walls non-uniformly.

7. The surface side sheet according to claim 6, wherein the fiber distribution of said surface side sheet is such that the standard deviation of a histogram as measured by "Formation Tester FMT-MIII," manufactured by Nomura Shoji Co., Ltd., is no less than 0.010 and no more than 0.060.

8. A surface side sheet for being used in an interlabial pad fitted between labia, comprising:

   a fiber aggregate wherein said fiber aggregate is a blend of two or more types of fibers which differ in fiber rigidity.

9. The surface side sheet according to claim 8, wherein said fibers which differ in fiber rigidity are a hydrophilic fiber of low fiber rigidity and a synthetic fiber of high fiber rigidity.

10. An interlabial pad comprising:

    a surface side sheet facing labial inner walls,
    a back face side sheet facing garments, and
    an absorbent body sandwiched between said surface side sheet and said back face side sheet and absorbing body fluids,

    wherein said surface side sheet is the surface side sheet according to any one of claims 1 to 9.

11. The interlabial pad according to claim 10, wherein an area of contact of said absorbent body with said surface side sheet is no more than 30% of an area of said absorbent body.

12. The interlabial pad according to claim 10 or 11, wherein a total size in a lateral direction of said surface side sheet in a state where the interlabial pad is folded in two along a folding line provided along a longitudinal direction so that said surface side sheet is set outside is longer than that in a state where the interlabial pad is flat.

13. The interlabial pad according to any one of claims 10 to 12, wherein the interlabial pad is for urine incontinence.

14. The interlabial pad according to any one of claims 10 to 12, wherein the interlabial pad is for vaginal discharge.

15. A method of improving adhesion of an interlabial pad to labial inner walls, said interlabial pad having a surface side sheet facing labial inner walls, a back face side sheet facing garments, and an absorbent body sandwiched between said surface side sheet and said back face side sheet and absorbing body fluids, comprising step of:

    forming said surface side sheet of a fiber aggregate wherein a binding force among fibers of said fiber aggregate is loose.

16. A method of lowering an area of coating of an adhesive agent for fitting an interlabial pad between labia, said interlabial pad comprising a surface side sheet facing labial inner walls, a back face side sheet facing garments, and an absorbent body sandwiched between said surface side sheet and said back face side sheet and absorbing body fluids, comprising step of:

    forming said surface side sheet of a fiber aggregate wherein a binding force among fibers of said fiber aggregate is loose.

# Fig. 1 A

# Fig. 1 B

# Fig. 2 A

# Fig. 2 B

# Fig. 3

# Fig. 4

# INTERNATIONAL SEARCH REPORT

International application No.

PCT/JP03/11064

A. CLASSIFICATION OF SUBJECT MATTER
Int.Cl$^7$ A61F13/511, 13/472, 5/455

According to International Patent Classification (IPC) or to both national classification and IPC

B. FIELDS SEARCHED

Minimum documentation searched (classification system followed by classification symbols)
Int.Cl$^7$ A61F13/511, 13/472, 5/455

Documentation searched other than minimum documentation to the extent that such documents are included in the fields searched

| | | | |
|---|---|---|---|
| Jitsuyo Shinan Koho | 1926–1996 | Toroku Jitsuyo Shinan Koho | 1994–2003 |
| Kokai Jitsuyo Shinan Koho | 1971–2003 | Jitsuyo Shinan Toroku Koho | 1996–2003 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2000-210334 A  (Uni-Charm Corp.),<br>02 August, 2000 (02.08.00),<br>& TW 453189 Y        & EP 20122007 A2<br>& CN 1263758 A | 9,10,15,16<br>1-5,12 |
| Y<br>A | WO 00/69484 A  (THE PROCTER AND GAMBLE CO.),<br>23 November, 2000 (23.11.00),<br>& JP 2003-527147 A | 9-11,15,16<br>1-5,12 |
| Y<br>A | JP 2001-137283 A  (Pigeon Corp.),<br>22 May, 2001 (22.05.01),<br>(Family: none) | 9-11,13-16<br>1-5,12 |

☒ Further documents are listed in the continuation of Box C.  ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| | |
|---|---|
| Date of the actual completion of the international search<br>17 November, 2003 (17.11.03) | Date of mailing of the international search report<br>02 December, 2003 (02.12.03) |
| Name and mailing address of the ISA/<br>Japanese Patent Office | Authorized officer |
| Facsimile No. | Telephone No. |

Form PCT/ISA/210 (second sheet) (July 1998)

**EP 1 547 559 A1**

## INTERNATIONAL SEARCH REPORT

International application No.

PCT/JP03/11064

| | C (Continuation). DOCUMENTS CONSIDERED TO BE RELEVANT | | |
|---|---|---|
| Category* | Citation of document, with indication, where appropriate, of the relevant passages | Relevant to claim No. |
| Y<br>A | JP 2001-309940 A (Daio Paper Corp.),<br>06 November, 2001 (06.11.01),<br>(Family: none) | 6,7,15,16<br>1-5,12 |
| Y<br>A | JP 2002-272786 A (Daio Paper Corp.),<br>24 September, 2002 (24.09.02),<br>(Family: none) | 6,7,13-16<br>1-5,12 |
| Y<br>A | JP 11-512943 A (The Procter & Gamble Co.),<br>09 November, 1999 (09.11.99),<br>& WO 97/46188 A | 6,7<br>1-5,12 |
| Y<br>A | CD-ROM of the specification and drawings annexed to<br>the request of Japanese Utility Model Application No.<br>298/1993(Laid-open No. 59039/1994)<br>(Sekisui Plastics Co., Ltd.),<br>16 August, 1994 (16.08.94),<br>(Family: none) | 6-9,13-16<br>1-5,12 |
| Y<br>A | JP 10-75978 A (Kao Corp.),<br>24 March, 1998 (24.03.98),<br>(Family: none) | 6-11,13-16<br>1-5,12 |
| Y | JP 2001-146663 A (Pigeon Corp.),<br>29 May, 2001 (29.05.01),<br>(Family: none) | 15,16 |
| Y<br>A | JP 2002-238947 A (Kao Corp.),<br>27 August, 2002 (27.08.02),<br>(Family: none) | 6,7,13-16<br>1-5,12 |
| Y<br>A | JP 2002-187228 A (Kao Corp.),<br>02 July, 2002 (02.07.02),<br>& EP 1209271 A1 & CN 1348026 A | 6-11,13-16<br>1-5,12 |
| A | JP 2002-65735 A (Uni-Charm Corp.),<br>05 March, 2002 (05.03.02),<br>& US 2002-26169 A1 & EP 1184021 A2<br>& AU 5800201 A & CN 1343484 A<br>& BR 105045 A | 1-16 |

Form PCT/ISA/210 (continuation of second sheet) (July 1998)

24

## INTERNATIONAL SEARCH REPORT

| International application No. |
|---|
| PCT/JP03/11064 |

**Box I    Observations where certain claims were found unsearchable (Continuation of item 2 of first sheet)**

This international search report has not been established in respect of certain claims under Article 17(2)(a) for the following reasons:

1. ☐ Claims Nos.:

because they relate to subject matter not required to be searched by this Authority, namely:

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

The inventions as set forth in claims 1 to 3 relate to the wet shear strength of a fibrous construct constituting a front face sheet, while the inventions as set forth in claims 4 and 5 relate to the tensile strength of a fibrous construct constituting a front face sheet. The inventions as set forth in claims 6 and 7 relate to the fiber distribution of a fibrous construct constituting a front face sheet, while the inventions as set forth in claims 8 and 9 relate to the structure of a fibrous construct constituting a front face sheet. Further, the inventions as set forth in claims 15 and 16 relate to the constraint force among fibers of a fibrous construct constituting a front face sheet. It appears that there is (continued to extra sheet)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

☒    No protest accompanied the payment of additional search fees.

Form PCT/ISA/210 (continuation of first sheet (1)) (July 1998)

| INTERNATIONAL SEARCH REPORT | International application No. |
|---|---|
| | PCT/JP03/11064 |

<u>Continuation of Box No. II of continuation of first sheet(1)</u>

no technical matter common to these groups of inventions.  Such being the case, this international application has 5 groups of inventions.

Form PCT/ISA/210 (extra sheet) (July 1998)